# EUROPEAN PATENT APPLICATION

(11) **EP 1 354 572 A2**
(43) Date of publication of application: **22.10.2003**
(21) Application number: 03252434.0
(22) Date of filing: 16.04.2003
(51) Int. Cl.: A61F 2/44, A61F 2/28

(54) **Implant and material for minimally invasive spinal interbody fusion surgery**

(30) Priority: 16.04.2002 US 123598
(71) Applicant: Tsou, Paul M., Santa Monica, California 90404 (US)
(72) Inventor: Tsou, Paul M., Santa Monica, California 90404 (US)
(74) Representative: Miller, James Lionel Woolverton

(57) **Abstract**

An implant device for spinal interbody fusion surgery has a shape substantially similar to a human excavated disc space, and includes a first modular end section (12), at least one modular middle section (15) disposed adjacent to the first modular end section (12), a second modular end section (13) disposed adjacent to the modular middle section (15) and wherein when the first end section (12), the middle section (15) and the second end section (13) are placed adjacent to each other, the implant device has a shape with is substantially oval, viewed from top, and a cross section which is bi-convex. The preferred embodiment of the implant device is manufactured from human bone material that has been formed into the shape of the modular components. Alternative embodiments are manufactured from non-human material such as hydrooxyapetite, ceramics, coral and other biodegradable material. Non-resorptable plastic and metal can be used as internal structural members of the implant modules.

## Description

### BACKGROUND

### 1. Field of the Invention

The present invention relates generally to percutaneous spinal interbody fusion surgery and, more specifically, to an implant design and material used to replace spinal nucleus pulposus when performing percutaneous endoscopic transforaminal lumbar and thoracic interbody fusion surgery.

### 2. Description of Prior Art

A substantial segment of the population suffers from spinal pain that is caused by degenerative, herniated or protruded intervertebral discs. Intervertebral discs are members of the spinal column that serve as cushions and mobile linkage elements between the adjacent vertebrae. The acute herniation of an intervertebral disc can lead to the compression of spinal nerve elements within the spinal canal and outside of the spinal canal. This can cause severe back pain, leg pain, muscle weakness, and possibly bowel and bladder dysfunction.

Traditional surgical methods to address the problem of spinal nerve element decompression include the transcanal methods of laminectomy or laminotomy. Optical aids such as microscopes, endoscopes or loupes are often used in these processes. The tissue retractor commonly used in this type of surgery is normally constructed with two blades. More recently, a tubular shaped retractor has been used. Traditionally, this procedure has required two to three days of hospitalization after completion of the surgery.

Chronic back pain due to disc failure, without dominant extremity symptoms, may also cause chronic functional impairment. Prior art solutions have surgically fused adjacent vertebrae together by placing bridging bone material from one vertebra above to one vertebrae below the symptomatic disc(s). The bone fusion surfaces may include the posterior vertebral elements. Sometimes, metal rods and screws have been used to stabilize the subject spinal fusion segment from the posterior approach. The intervertebral bone end-plates are also important fusion surfaces that interact with an implant to bond together adjacent vertebrae. Currently available intervertebral fusion implants have configurations of cylinders, blocks or chips and consist of metallic, carbon fiber, hydrooxyapetite, coral bone, etc. As necessary, the intervertebral space may be approached posteriorly or anteriorly. Additionally, a combined anterior and posterior approach is sometimes used. Depending upon the circumstances, the procedure may be accomplished in one operation or may require several staged operative procedures.

These prior art techniques have typically caused significant access tissue trauma, even when the skin incision was reduced in length. One result of greater access tissue trauma is a longer healing and rehabilitation period. Another result is the potential for complications accompanying or following the surgery.

With new endoscopic percutaneous transforaminal techniques, a surgeon can operate through a smaller (roughly 8-16 millimeter) opening using endoscopic viewing instruments and miniaturized tools. Because the newer techniques cause less destabilization and access surgical trauma, rehabilitation begins earlier and is of significantly shorter duration.

In clinical situations where a patient suffers from lumbar chronic discogenic pain or low-grade instability, a fusion operation is often offered to mitigate pain and functional impairment. The preferred surgery techniques adopt an extraspinal canal minimally invasive technique. To fuse adjacent vertebrae, implant material is placed in the evacuated disc space between the bony end-plate of the target vertebrae. After insertion of the structural implant material and any additional non-structural osteogenic agents, ingrowth of new autologous bone gradually replaces the implant to create a unified structure that includes the first and last vertebrae in the fusion segment. Prior art implant material included structural angular bone blocks, metallic cages, carbon fiber blocks or bone chips. Prior art laparoscopic anterior lumbar fusion technique uses cylindrical metallic cages or bone dowels.

Although an improvement over earlier techniques, these prior art devices have several problems. Significantly, the prior art cylindrically shaped implants do not achieve maximum surface contact with the generally flat surface of the host end plate bed. Thus, seating of cylindrical/round shaped fillers requires some end-plate cutting. Surgical end-plate cutting structurally weakens the end-plate and introduces the risk of metallic fillers settling into the softer vertebral cancellous body.

One specific prior art technique is discussed in United States Patent No. 6,217,509, which describes an access tubular channel from the skin to the targeted work area of the lamina (an approach that is typically used in the posterior transcanal spinal approaches). The working channel inside the tube allows for the use, as needed, of a viewing element, operating tools, tissue retractors, suction channels and a fluid channel. This prior art method is problematic when used in either its preferred embodiment or any other approach because it has no annular docking mechanism to ensure the safe delivery of the implant from outside of the body to the intervertebral disc space.

Other prior art uses hydrogel "nucleus replacement" implants. Nucleus replacement means only the soft jelly part of the disc is replaced. The hydrogel needs a jacket for containment and has no effective fixation to the bone end-plate or the annulus. Additionally, clinical trials outside of the United States to date demonstrate the potential for the hydrogel to extrude from the jacket and disc space.
Therefore, what is needed is an improved implant material for spinal interbody fusion surgery that can be implanted into an intervertebral disc space using the preferred minimally invasive percutaneous transforaminal endoscopic spinal surgery methodology or the traditional approach methods.

### BRIEF SUMMARY OF THE INVENTION

The present invention overcomes the limitations of the prior art by providing an implant material for spinal surgery, which can be used with a minimally invasive surgical technique. Such an implant device has a shape substantially similar to a human disc space after evacuation of nucleus pulposus and cartilaginous end plate. In order to insert the implant through minimally invasive procedures, the whole implant is modularized. The implant consists of a first modular outer section, at least one modular middle section and a second modular outer section. When the first outer section, the middle section and the second outer section are placed adjacent to each other, the implant device has a shape which is substantially oval and a cross section which is bi-convex. The preferred embodiment of the implant device is manufactured from human bone material or any biodegradable material, which has been formed into the shape of the modular components. Inert plastic and metal can be used as implant internal structural support.

The present invention is a substantial improvement over the prior art. Because of its modularity, the bone graft material of the present invention can be easily be inserted into position using minimally invasive surgical techniques. The present invention further permits the use of an implant which fills the entire evacuated disc space for maximum graft to host bed surface contact. Additional features and advantages of the present invention will be appreciated by reviewing the following drawings and detailed description of the invention.

### BRIEF DESCRIPTION OF THE DRAWINGS

Figure 1 is an idealized side view of a human spinal column showing the arrangement of vertebrae and discs.
Figure 2 is a cutaway view showing the placement and structure of discs in the spinal column.
Figure 3 is a top view of a human vertebra showing a disc axial cross section.
Figure 4 is a perspective view of the implant device of the present invention.
Figure 5 is a plan view of the modular elements of the implant device.
Figure 6 shows adjacent vertebrae with the disc removed, and illustrates the placement of the implant of the present invention.
Figure 7 illustrates alternative arrangements of the modular elements of the present invention.
Figure 8 illustrates the anatomy of the L₅-S₁ disc in a human spinal column.
Figure 9 is a cross sectional view of the implant taken along the line 6-6 in Figure 5.
Figure 10 is a cross sectional view of an alternative embodiment of the implant which is used to replace the L₅₋S₁ vertebra.
Figure 11 illustrates several different alternative embodiments of the invention.
Figure 12 illustrates an alternative embodiment where only a portion of the implant is placed within the intervertebral space.
Figure 13 illustrates the end plate of a human vertebra.
Figure 14 illustrates an alternative embodiment of the implant.
Figure 15 shows the insertion of the implant into the intervertebral space using an anterior surgical method.
Figure 16 illustrates the insertion of the implant of the present invention using a transforaminal minimally invasive surgical technique.
Figure 17 illustrates several different alternative embodiments of the invention.

### DETAILED DESCRIPTION OF THE INVENTION

According to the present invention, there is disclosed an implant device used when performing percutaneous spinal transforaminal endoscopic interbody fusion. In the following description, for the purposes of explanation, specific devices, production details, surgical methods and tools are set forth in order to provide a more thorough understanding of the invention. It will be apparent to those skilled in the art, however, that the present invention may be practiced without these specifically enumerated details and that the preferred embodiment can be modified so as to provide other capabilities. In some instances, well-known structures and methods have not been described in detail so as not to obscure the present invention unnecessarily.

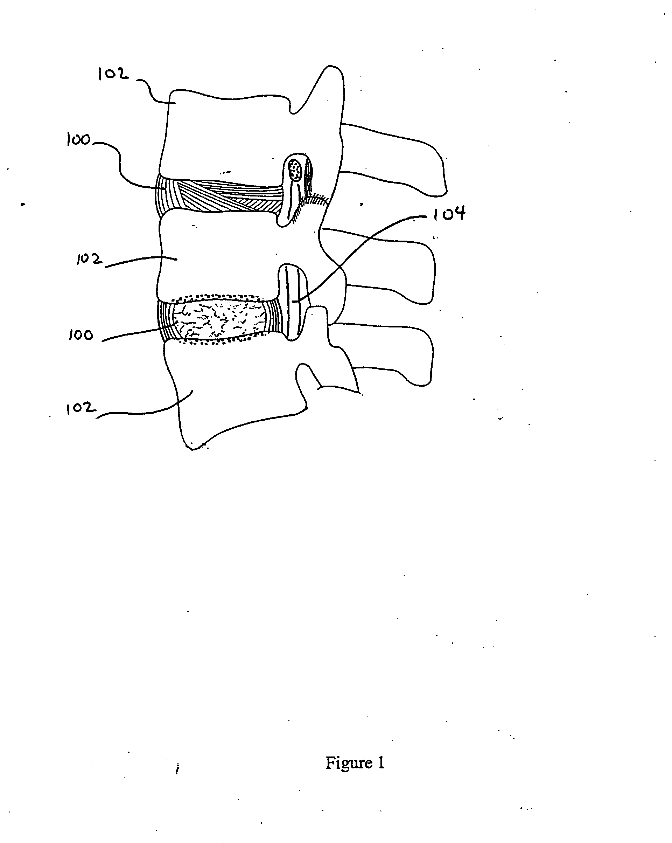

Referring first to Figure 1, an idealized side view of a human spinal column illustrating discs located between adjacent vertebrae is shown. A disc 100 is located between each of the adjacent vertebrae 102. The dural sac 104 is shown passing vertically through the spinal canal. As shown, there is one disc located between paired adjacent vertebrae.

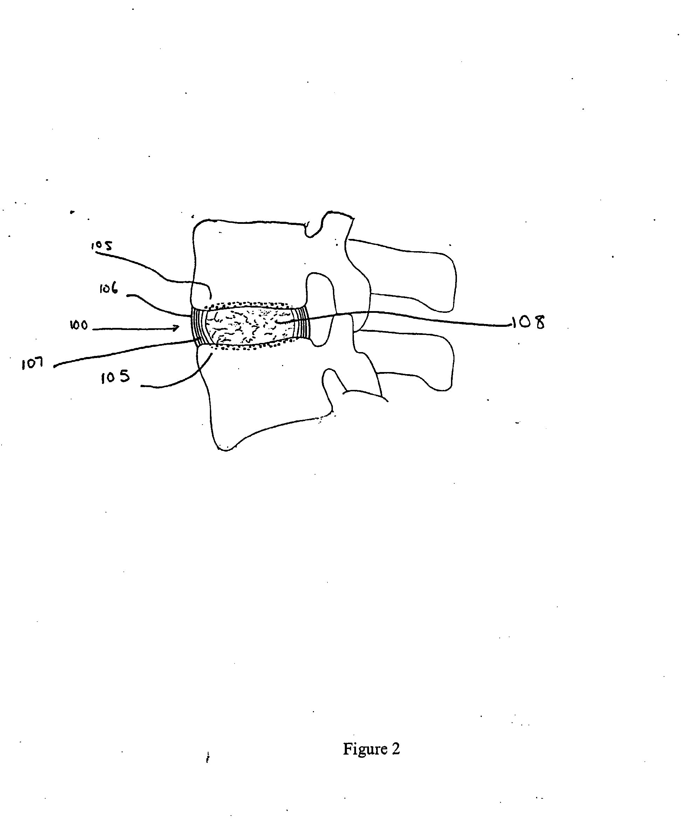

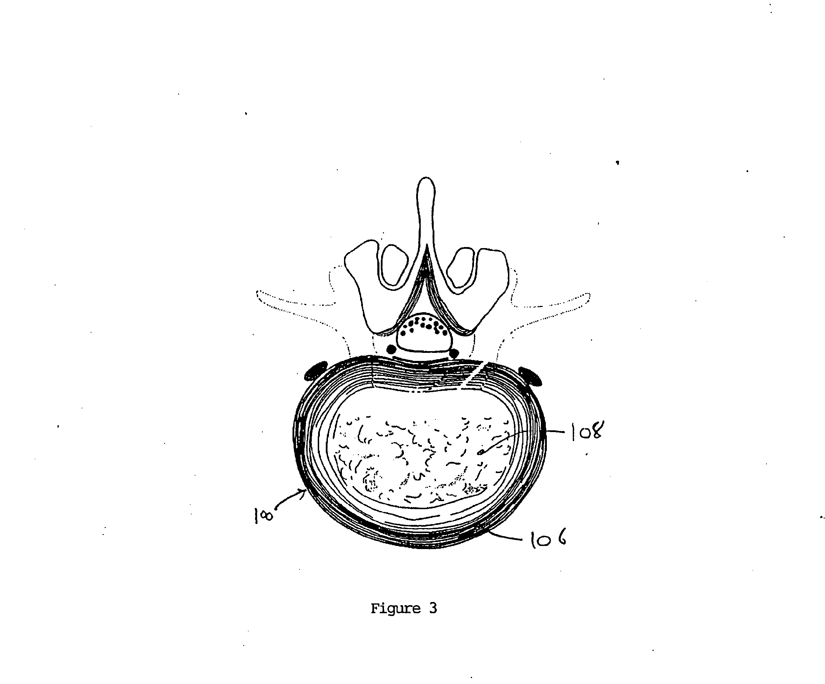

Figure 2 illustrates a cut-away view of a single disc between the vertebrae. The disc 100 consists of a cartilaginous end plate 105 as its top (cranial) and bottom (caudad) surface. The edge of the disc is surrounded by tissue known as the annulus fibrosus 106. The annulus fibrosus is composed of structured layers of fibrous tissue and fibro-cartilage. The layers 107 are arranged concentrically one within the other. The interior of the disc is filled with pulposus 108, a proteoglycan of gelatinous consistency. Figure 3 is a schematic axial view, which transects a human disc 100 located on top of a vertebra 102. The annulus fibrosus 106 and pulposus 108 are shown in this Figure.

The shape and size of the discs can vary in different parts of the spine. In shape, they accurately correspond to the surfaces of adjacent vertebrae. The discs are substantially oval in the cervical and lumbar regions of the spine. In the thoracic region, the discs have a more circular shape. The thickness of the discs may also vary in different regions of the body. The size of the discs is greatest in the lumbar region. Figures 1 through 3 illustrate the typical anatomy associated with human discs. It will be apparent to those of skill in the art, however, that normal variations in this anatomy may arise in the human species.

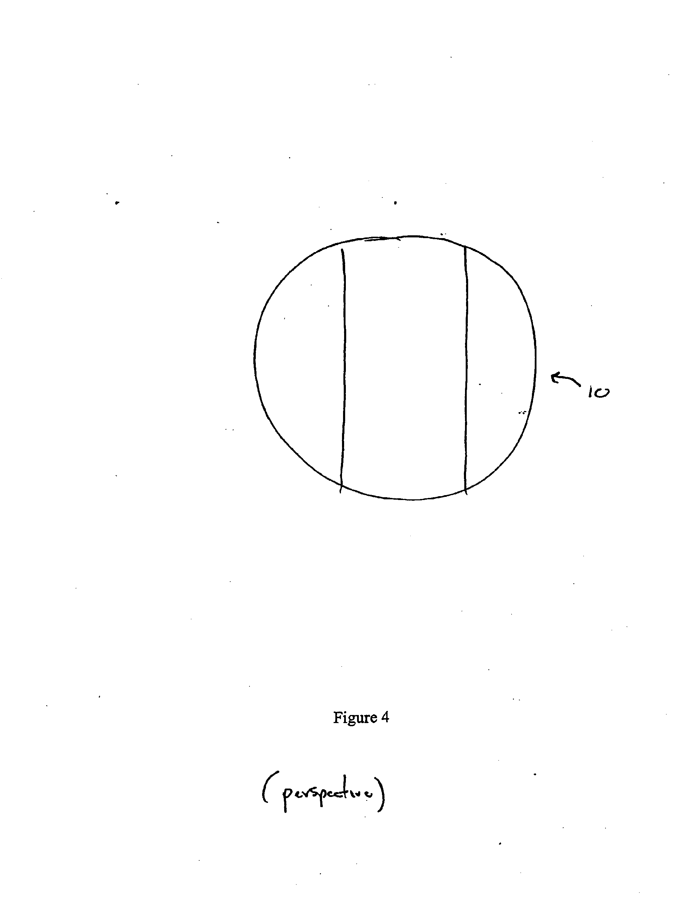

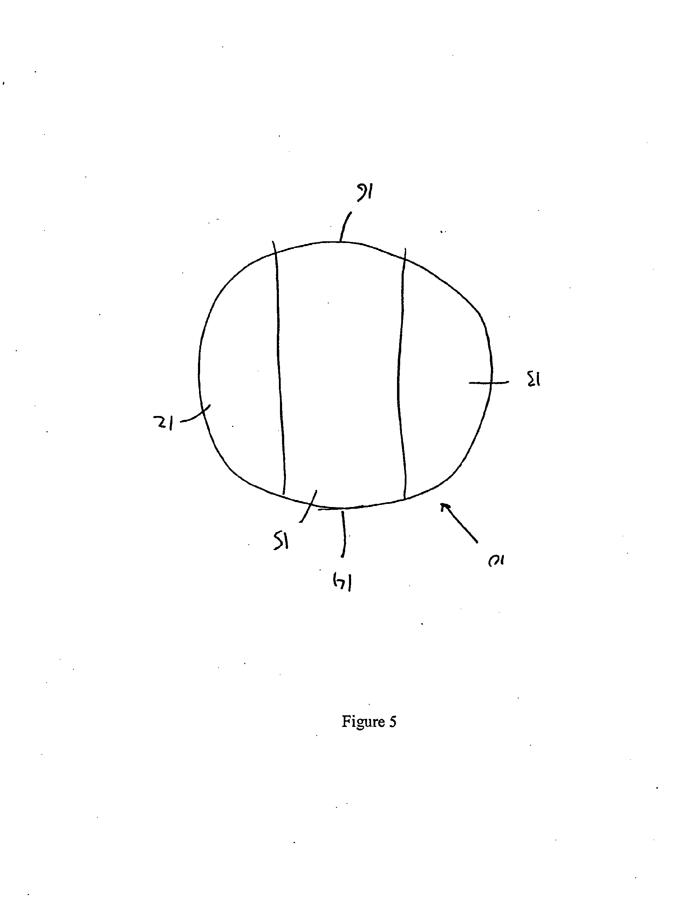

Referring next to Figures 4 and 5, the preferred embodiment of the implant 10 of the present invention is illustrated. Figure 4 is a perspective view of the implant and Figure 5 is a plan view looking at the top surface of the device. The implant 10, as shown, has a shape which, generally conforms to the space between adjacent osseous vertebrae. The implant 10 is inserted into the space between the vertebrae after the disc nucleus and cartilaginous end plate have been evacuated in a surgical procedure. Typical reasons for removal of the disc are discussed above. However, it will be apparent that the present invention can be used in any instance when it is necessary to fuse adjacent vertebrae.

The overall shape of the implant is best illustrated in Figure 5, which is the plan view of the device. When viewed from above, the implant is generally oval in shape. The implant 10 has two end sections 12, 13 that are curved. At least one middle section 15 is located between the two ends. The periphery of the implant is generally curved. Both sides 14 & 16 of the preferred embodiment of the implant 10 are convex in curvature.

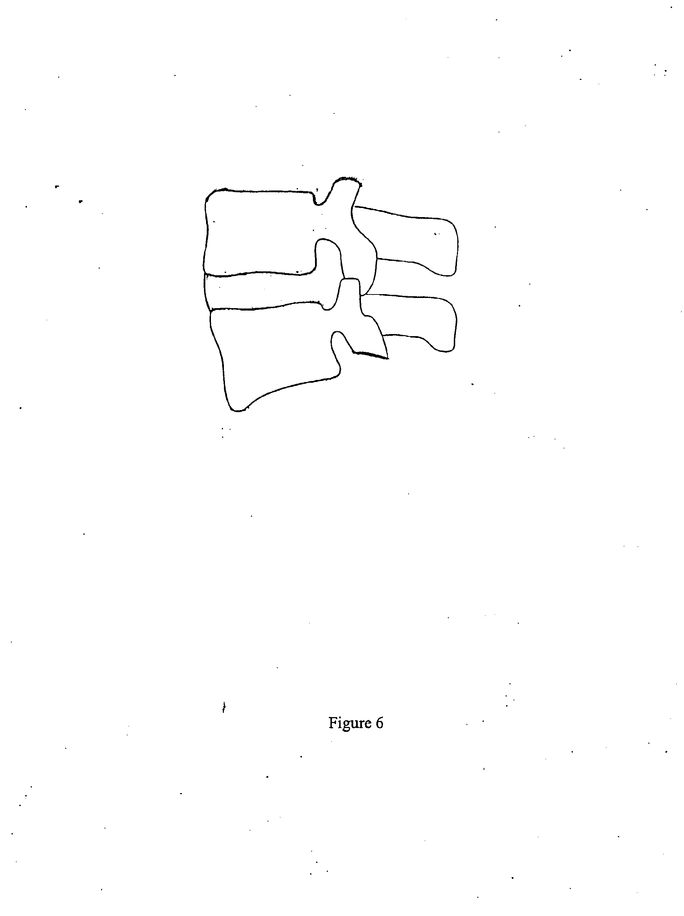

The implant in the preferred embodiment is of a size and shape substantially equal to the shape of an evacuated disc space. This space is illustrated in Figure 6. Figure 6 shows adjacent vertebrae 102 with the disc 100 that is normally between them removed. The location of the implant 100 is shown by the dashed line in Figure 6. The preferred embodiment of the whole implant has a length, width and height of approximately 22-28mm x 18-24mm x 8-16mm. It will be apparent to those of skill in the art that the implant may be manufactured in a range of sizes, and that it is not limited to this particular size. For example, female patients will quite often require that an implant of smaller dimensions be used as compared to male patients. Patients with unusual or atypical anatomy may require implants which, vary greatly in size from the average implant dimensions.

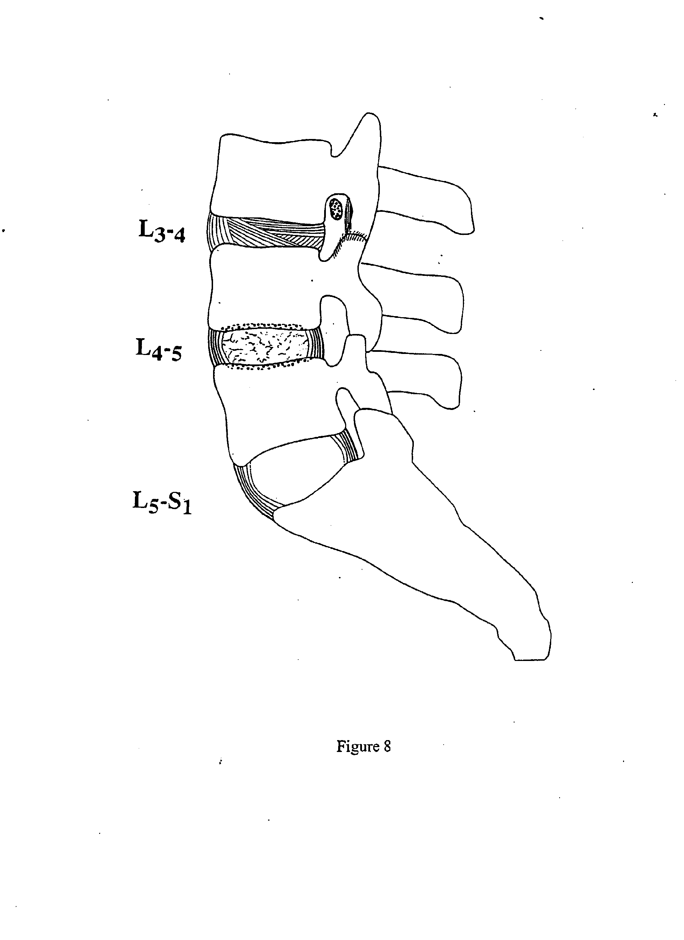

It is anticipated that the present invention may be used to replace any of the discs, which are present in the human body. The disc, which is known as the L₅₋S₁ disc has a shape, which is generally different from the other discs in the human body. The L₅₋S₁ disc is illustrated in Figure 8. The L₅₋S₁ disc is the lowest disc in the spinal column. As can be seen, the anterior height of the L₅₋S₁ disc is greater than its posterior height. This feature is known as a height variant, anterior in this example. The present invention will accommodate any disc spaces that have a disc height variant in any section of a disc.

The implant 10 is made of modular components. Referring again to Figures 4 and 5, the modular nature of the implant is illustrated. Several different components fit together to make up the whole implant. Figure 5 illustrates that the preferred embodiment of the implant includes three components. The individual modular components of the implant 10 have rounded edges facing the inner walls of the annulus fibrosus (not shown in Figure 5) and generally flat surface facing each other. From the top view, the components are separately either half moon (elements 12 and 13) or generally rectangular (element 15) in shape.

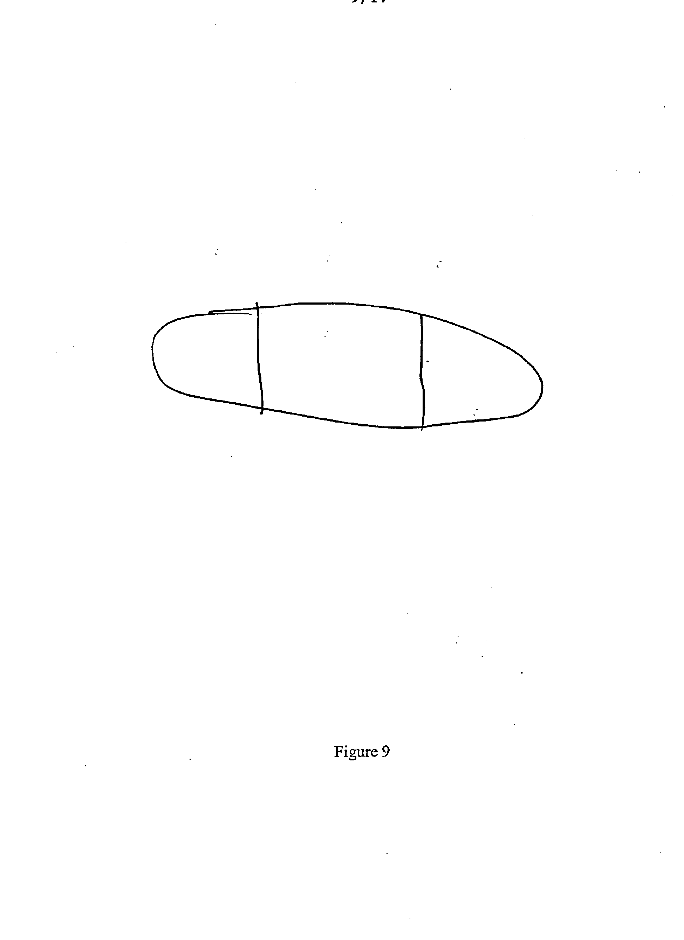

The implant is bi-convex in cross-section. Figure 9 illustrates a cross-sectional view of the implant 10 taken along the line 9-9 in Figure 5. The implant will have similar cross-sectional shape in other directions. The bi-convex shape is an important aspect of the present invention. This shape permits the implant to have total surface contact with host bed as compared to the prior art cylinder and block shaped grafts.

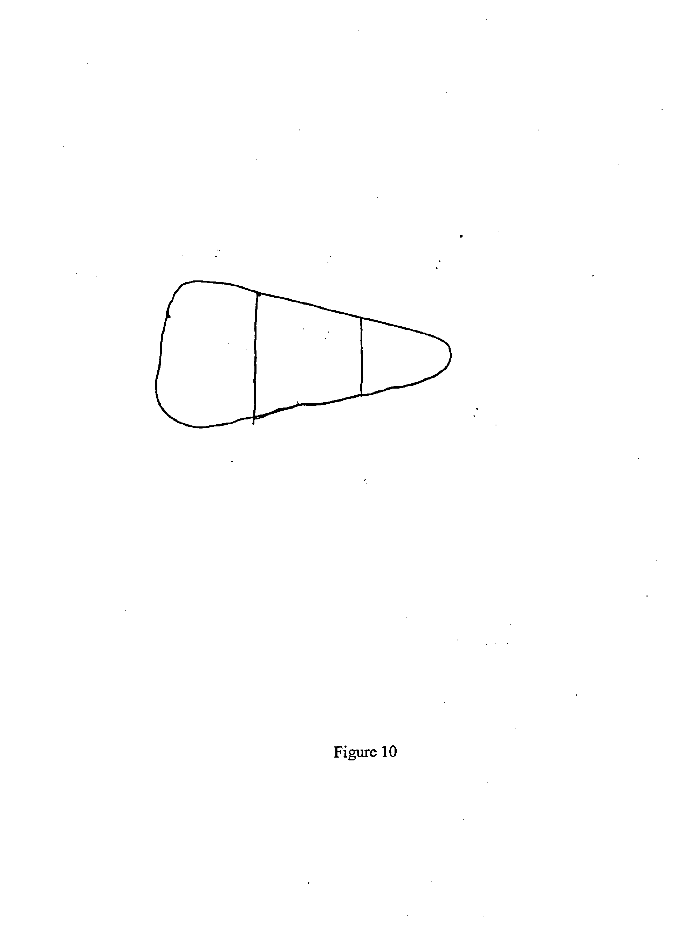

As noted above, the L₅-S₁ disc has an anterior height variant. The present invention accommodates this variant in an alternative embodiment. This alternative embodiment is illustrated in Figure 10, which is a cross section of the implant. As shown, one end of the implant is thicker than the other. The implant is shaped to generally conform to the shape of the L₅₋S₁ disc.

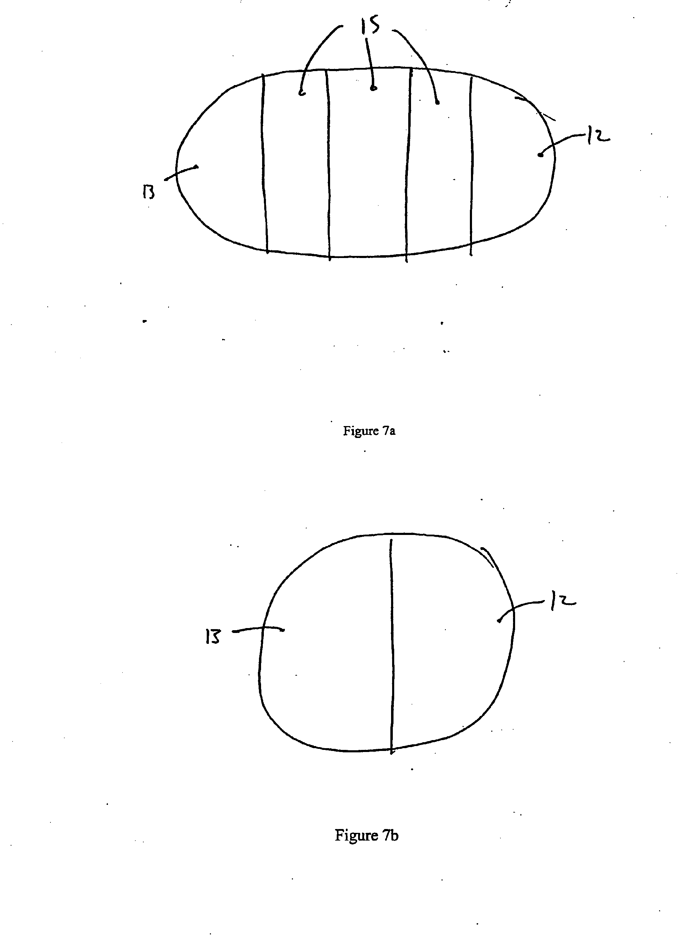

It will be apparent that a larger number of middle sections 15 can be used to make up the implant 10. Figure 7a illustrates an alternative embodiment of the invention, which includes 3 separate middle sections 15 between the end sections 12 and 13. Alternatively, the middle section 15 can be eliminated entirely. Although the preferred arrangement of the modular sections is shown in Figure 5, the sections can take on different shapes and arrangements without departing from the scope of the present invention. The number of middle sections that are used will depend on different factors. These primarily include the width of the middle sections 15 and the size of the end plates of the adjacent vertebrae.

The implant in the preferred embodiment is manufactured from human bone material. The preferred embodiment uses donor bone. The modular sections can be carved from single pieces of bone. Alternatively, the sections can be made from bone dust or flakes, which is formed and pressed into shape. Other osteoinductive and osteoconductive materials can also be used. Examples of such materials include ceramic, hydrooxyapetite, coral, etc. Plastic or metal can be incorporated as internal structural elements for strength.

The implant is manufactured from bone material in order to permit the implant to fuse with the end plates of the vertebrae. The osteoinductive and osteoconductive materials have a similar effect. In some clinical instances, however, it may not be advantageous for the implant to bond to the end plates. In such cases, the implant can be manufactured entirely from an inert material. In most instances when the implant is manufactured from an inert material, the implant acts as a temporary place holder. The inert implant can be replaced later with the preferred embodiment or other devices.

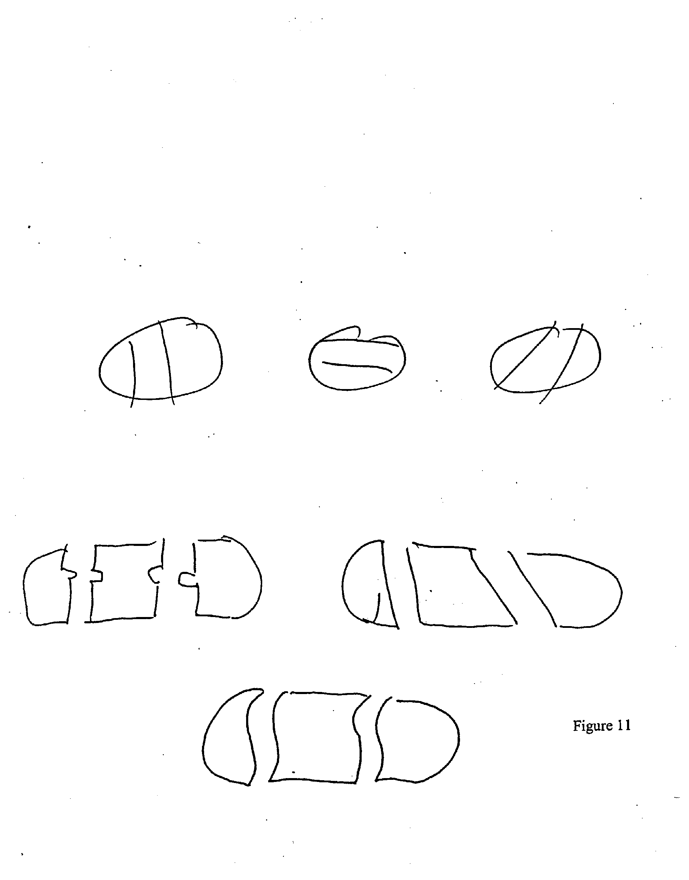

Referring again to Figure 5, it is seen that in the preferred embodiment, implant is separated into modular pieces by cuts that are oriented in a front-to back direction. It will be apparent to those of skill in the art, however, that the cuts can be oriented in ways that are different from the preferred embodiment. Figure 11 illustrates a few of the different ways that the implant can be cut into modular pieces. For example, the cuts can be disposed in a lateral, or side-to side orientation as shown in Figure 11a. Alternatively, the cuts can be on the oblique, as is shown in Figure 11b. Referring again to Figure 5, The modular sections 12, 13, 15 generally have straight side edges. (See Figure 9). It may be advantageous, however, to incorporate grooved, beveled or serrated edge in order to limit movement between the modular components. Examples of such edges are shown in Figure 11c.

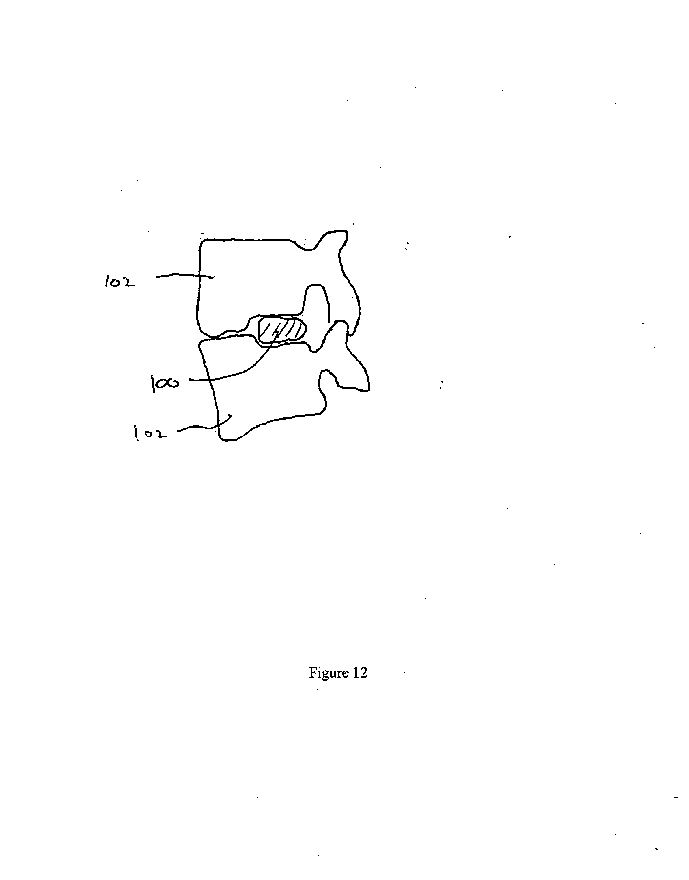

The foregoing discussion has assumed that the present invention will be used in cases where a disc will be completely removed, and replaced by an implant. In a few instance, however, it is not desirable or necessary to replace the entire disc. In other cases, anatomical limitations of the patent make it impossible to place the entire implant between adjacent vertebrae. For example, a congenital defect or deformity may require only a small portion of the inter-vertebral space being fused. The modular nature of the present invention permits only a portion of the overall implant to be inserted between adjacent vertebrae. Figure 12 illustrates a situation where only approximately half of the typical intervertebral space has been filled with the implant, fusion of the remainder of the space being inaccessible or unnecessary due to an atypical shape of the bone.

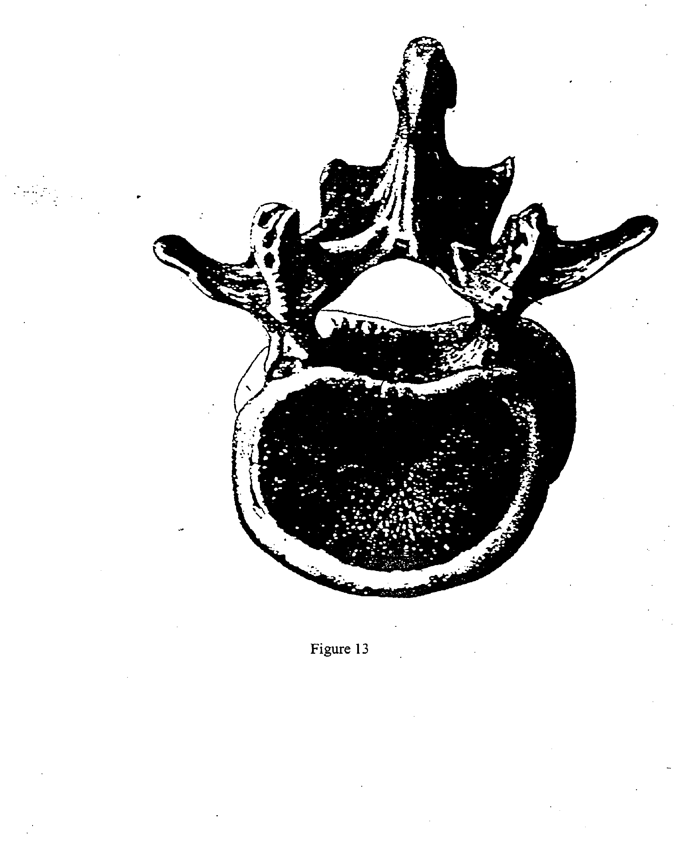

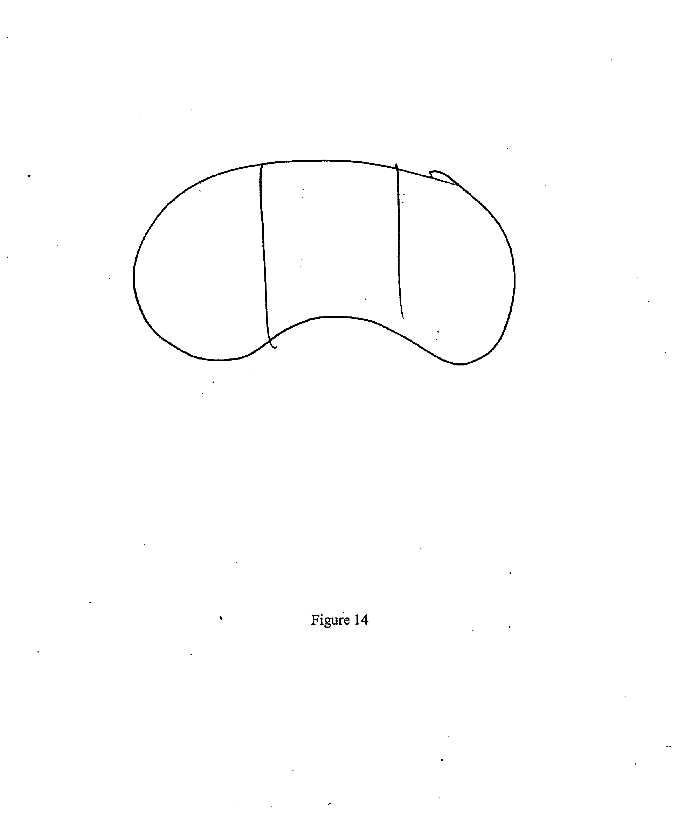

Figures 1-3 illustrate the typical anatomy of vertebrae and discs. In some instances, the end plates of the vertebrae may have a shape, which is not entirely oval. A sample of one such vertebra is shown in Figure 13. It is seen that the end plate is not entirely oval, but rather resembles a kidney bean. One side of the oval is concave. The present invention accommodates these vertebrae in an alternative embodiment. The alternative embodiment is shown in Figure . As can be seen, one edge of the implant is also convex, to conform to the shape of the vertebra illustrated in Figure 14.

The implant design of the present invention is used most advantageously in connection with a method of percutaneous transforaminal endoscopic lumbar interbody fusion surgery. One such method of surgery is described in Patent Application Serial No. 09/997,361 and is briefly reviewed here. The method of percutaneous transforaminal endoscopic lumbar surgery is the most desired method of surgery because, among other reasons, it is least invasive. It will be apparent to those of skill in the art that the implant of the present invention can be utilized with different surgical methods and approaches, including methods such as the traditional posterior approach, or the anterior approach, which are all known in the prior art.

With the method of percutaneous transforaminal endoscopic lumbar surgery, the general method of access utilizes a system of cannulae to create a tunnel from an opening in the skin window to the target area. The target area is generally the painful disc to be removed. The initial step is to insert a large bore guide needle following the preferred method from the skin window to the foraminal annular window. The subsequent steps require the exchange of guide pin, cannulated obturator and progressively larger beveled cannulae.

A series of progressively larger cannulae are then exchanged within the annular window. At all times the delivery system is docked within the annular window. Nucleus pulposus and cartilaginous end plates are removed utilizing appropriate tools. The round shaped opening in the annular window is changed into rectangular shape by next inserting a round shaped spreader then exchanged to a flat blade spreader. The final device of the delivery system that comprises the tunnel walls is the flat blade spreader and its covers. The generally angular shaped tunnel permits transit of mostly angular shaped implant module without risk of entanglement in the soft tissue.

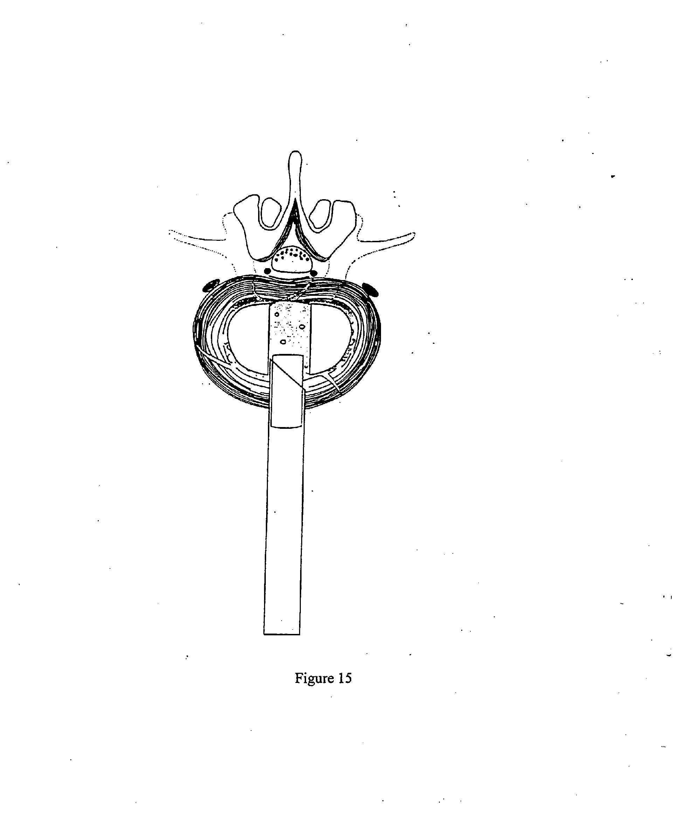

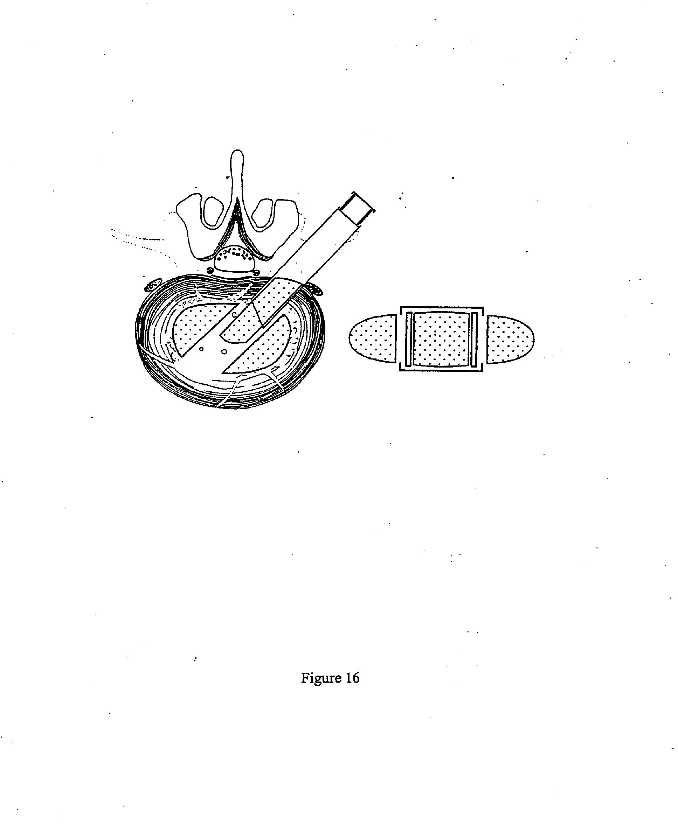

The sizing of the whole implant is determined by measuring the dimensions of the excavated cavity under direct endoscopic visualization. After completion of the above preparations, introduction of the optimally sized modular disc-shaped implant components from the outside of the skin surface into the disc space is performed. The preferred method uses at least two pieces of modular disc-shaped implant components. Each piece has at least one flat surface. The flat surface of the subsequently implanted piece opposes and engages the flat surface of the last inserted module. The first piece is inserted and pushed as far away as possible to the right or left side of the delivery tube trajectory against the inner surface of the annulus. Gradual seating of the components is made possible by using various contoured impactors and spreaders. Once the first component is in place, the second component is inserted to the opposite side of the tube trajectory. For a disc space of larger size, a third, fourth or more implant component, rectangular in shape, can be introduced between the right and left positioned components. Figure 15 shows the implant being inserted using an anterior surgical approach. Figure 16 illustrates an implant, which is cut on the obliquely being inserted into place.

It is desirable for the implant to have a large height and cross-sectional surface area to maximize the contact area with the opposing host end-plates. When the implant material is in place it will place the end-plate and graft surfaces under firm compression. Since the tunnel from the skin window into the disc space is very limited in height and width, it is preferred to make each modular element of the implant just small enough to fit through the delivery tube. Two or more components of the whole implant make separate transit through the relatively smaller tube. The modular nature of the implants allows the implant materials to be positioned in the intervertebral area with minimally invasive techniques. As noted above, there is a direct relationship to the degree of invasiveness required in a surgical procedure and the recovery time necessary after the surgery. When there is minimal access trauma and minimal destabilization, there is no need for additional stabilization surgery. Any necessary rehabilitative activities can be started much sooner after the conclusion of the surgery.

After the insertion of the implant material, supplementary osteoconductive and osteoinductive agents in the form of paste, jelly or sponge can also be inserted to fill any small crevices or voids that remain in the target intervertebral disc space.

The present invention has been described as being used to replace nucleus pulposus and cartilaginous end plate in surgery on human patients. It will be apparent to those of skill in the art that the present invention can be easily modified to be used in a wide variety of different animals with equal effectiveness. Therefore, the present invention will have an application and similar efficacy in the veterinarian arts as well.

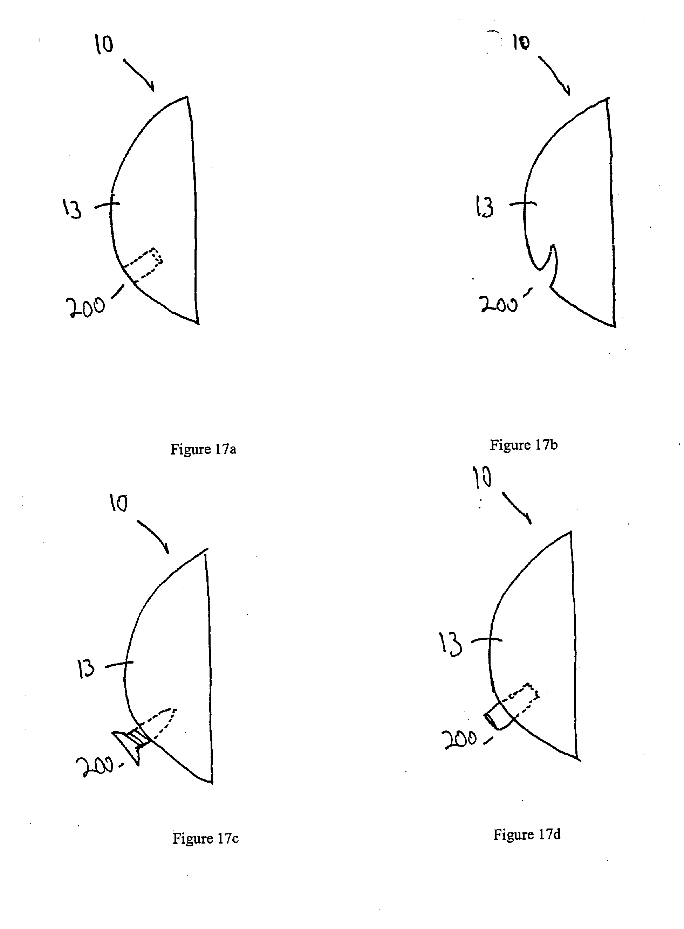

An applicator device can be used to apply the modular section. Such an applicator device will contain a means for holding and exercising control over the invention. Applicator means can be, but are not limited to, tweezers, pliers, screwdrivers, socket wrenches or handgrips. An alternative embodiment of the invention will include an attachment means, which is incorporated into or onto the modular section that is adjusted to be engaged by the applicator. The attachment means will be designed so as to allow the operator a greater deal of control over the modular section when using the applicator device to insert the modular section between the vertebrae. The attachment means 200 can be, for example, a hole, a notch, a screw, or a peg. Figure 17 illustrates several examples of this alternative embodiment. It will be obvious to those skilled in the art that this list of various attachment means is merely illustrative and non-exhaustive.

It will be apparent to those skilled in the art that the foregoing description is for illustrative purposes only, and that various changes and modifications can be made to the present invention without departing from the overall spirit and scope of the present invention. The full extent of the present invention is defined and limited only by the following claims.

## Claims

**1.** An implant device for spinal interbody fusion surgery, comprising:
a plurality of modular elements, said elements being adapted to fit together into a single assembly, such that said assembly has a shape substantially similar to an excavated disc space.

**2.** The device of Claim 1 wherein said implant device is substantially oval in shape.

**3.** The implant device of Claim 1 wherein said assembly is bi-convex in cross-section.

**4.** The implant device of Claim 1 where said implant device is manufactured from biodegradable material.

**5.** The implant device of Claim 1 wherein said modular elements include two outer members and a plurality of inner members wherein said implant is cut laterally into said members from a unitary element.

**6.** The implant device of Claim 1 wherein said modular elements include two outer members and a plurality of inner members wherein said elements are cut obliquely into said members from a unitary element.

**7.** The implant device of Claim 1 wherein said implant device elements include two outer members and a plurality of inner members wherein the interior edge of the two said outer members and the lengthwise edges of the said plurality of inner members are cut in a non-straight line such that the edge of one said member couples with the edge of the adjacent said member when said implant is fully assembled.

**8.** The implant device of Claim 1 wherein said implant device is manufactured from carbon fiber material.

**9.** The implant device of Claim 1 wherein said implant device is manufactured from hydrooxyapetie material.

**10.** The implant device of Claim 1 wherein said implant device is manufactured from autologous bone material.

**11.** The implant device of Claim 1 wherein said implant device is manufactured from donor bone material.

**12.** The implant device of Claim 1 wherein said implant device is manufactured from cylinders made from materials that include at least one of a group including metallic, carbon fiber, hydrooxyapetie, donor bone material or autologous bone material.

**13.** The implant device of Claim 1 wherein said implant device is manufactured from blocks made from materials that include at least one of a group including metallic, carbon fiber, hydrooxyapetie, donor bone material or autologous bone material.

**14.** The implant device of Claim 1 wherein said implant device is manufactured from chips made from materials that include at least one a group including metallic, carbon fiber, hydrooxyapetie, donor bone material or autologous bone material.

**15.** The implant device of claim 1 wherein said implant device is manufactured from flakes of human bone which have been formed into each of said modular section.

**16.** The implant device of claim 1 wherein said implant device is manufactured from dust of human bone which has been formed into each of said modular sections.

**17.** The implant device of Claim 1 wherein said implant device is manufactured from coral bone material.

**18.** The device of Claim 1 wherein said implant device has one side that is substantially concave in shape.

**19.** The device of Claim 1 wherein said implant device has one edge that is substantially convex in shape.

**19.** The implant device of Claim 1 where said implant device is manufactured from an inert material.

**20.** The implant device of Claim 1 wherein said implant is formed such that a first end of said implant device is thicker than a second end of said implant device such that when said device is fully assembled, the height of said module tapers from said first end to said second end.

**21.** An implant device for spinal surgery, said device having a shape substantially similar to an excavated disc space healthy human disc, comprising:
a first modular end section;
at least one modular middle section disposed adjacent to said first modular end section;
a second modular end section disposed adjacent to said at least one modular middle section;
wherein said first end section, said at least one middle section and said second end section are placed adjacent to each other, such that said implant device has a shape with is substantially oval, and one side of said oval is concave, and a cross section which is bi-convex.

**22.** The implant device of Claim 21 wherein said first modular end section; is thicker than said second modular end section

**23.** The implant device of claim 21 wherein said implant device is manufactured from flakes of human bone which have been formed into each of said modular sections.

**24.** The implant device of claim 21 wherein said implant device is manufactured from dust of human bone which have been formed into each of said modular sections.

**25.** The device of claim 21 wherein each of said modular sections is manufactured from an inert material.

**26.** The implant device of Claim 21 wherein said implant device comprises two outer members and a plurality of inner members wherein said implant is cut laterally into said members.

**27.** The implant device of Claim 21 wherein said implant device comprises two outer members and a plurality of inner members wherein said implant is cut obliquely into said members.

**28.** The implant device of Claim 21 wherein said implant device comprises two outer members and a plurality of inner members wherein the interior edge of the two said outer members and the lengthwise edges of the said plurality of inner members are cut in a non-straight line such that the edge of one said member couples with the edge of the adjacent said member when said implant is fully assembled.

**29.** The implant device of Claim 21 wherein said implant device is manufactured from carbon fiber material.

**30.** The implant device of Claim 21 wherein said implant device is manufactured from hydrooxyapetie material.

**31.** The implant device of Claim 21 wherein said implant device is manufactured from autologous bone material.

**32.** The implant device of Claim 21 wherein said implant device is manufactured from donor bone material.

**33.** The implant device of Claim 21 wherein said implant device is manufactured from cylinders made from materials from a group including at least one of metallic, carbon fiber, hydrooxyapetie, donor bone material and autologous bone material.

**34.** The implant device of Claim 21 wherein said implant device is manufactured from blocks made from materials from a group including at least one of metallic, carbon fiber, hydrooxyapetie, donor bone material and autologous bone material.

**35.** The implant device of Claim 21 wherein said implant device is manufactured from chips made from materials from a group including at least one of metallic, carbon fiber, hydrooxyapetie, donor bone material and autologous bone material.

**36.** The implant device of claim 21 wherein said implant device is manufactured from flakes of human bone which have been formed into each of said modular section.

**37.** The implant device of claim 21 wherein said implant device is manufactured from dust of human bone, which has been formed into each of said modular sections.

**38.** The implant device of Claim 21 wherein said implant device is manufactured from coral bone material.

**39.** The device of Claim 21 wherein said implant device has one side that is substantially concave in shape.

**40.** The device of Claim 21 wherein said implant device has one edge that is substantially convex in shape.

**41.** The implant device of Claim 21 where said implant device is manufactured from an inert material.

**42.** The implant device of Claim 21 wherein said implant is formed such that a first end of said implant device is thicker than a second end of said implant device such that when said device is fully assembled, the height of said module tapers from said first end to said second end.

**43.** A method of surgically replacing a human nucleus pulposus and cartilaginous end plate comprising the steps of:
(a) creating a tunnel in soft tissue of a patient, said tunnel linking a skin window of said patent's skin with said disk to be replaced;
(b) removing said human nucleus pulposus from between adjacent vertebrae;
(c) removing any additional material located between said bony adjacent vertebrae;
(d) inserting at least one tubular system into said tunnel;
(e) passing at least two modular components of a spinal implant through said tube into a space previously occupied by said disc, each of said modular components being separately passed through said tube and into the appropriate location.

**44.** Apparatus for surgically replacing a human nucleus pulposus and cartilaginous end plate comprising:
(a) means for creating a tunnel in soft tissue of a patient, said tunnel linking a skin window of said patient's skin with said disk to be replaced;
(b) means for removing said human nucleus pulposus from between adjacent vertebrae;
(c) means for removing any additional material located between said bony adjacent vertebrae;
(d) means for inserting at least one tubular system into said tunnel; and
(e) means for passing at least two modular components of a spinal implant through said tube into a space previously occupied by said disc, each of said modular components being adapted to be separately passed through said tube and into the appropriate location.
